# FASCICULE DE BREVET EUROPEEN

(11) **EP 0 436 426 B1**
(45) Date de publication et mention de la délivrance du brevet: **15.06.1994**
(21) Numéro de dépôt: 90403725.6
(22) Date de dépôt: 21.12.1990
(51) Int. Cl.: C07D 233/74, C07D 233/72

(54) **Nouveau procédé de préparation de dérivés de la 1-phénylimidazolin 2,5-dione**
Verfahren zur Herstellung von 1-Phenylimidazolin-2,5-dion-Derivate
Process for the preparation of 1-phenylimidazolin-2,5-dion derivatives

(30) Priorité: 22.12.1989 FR 8917046
(43) Date de publication de la demande: 10.07.1991
(73) Titulaire: ROUSSEL-UCLAF, 75007 Paris (FR)
(72) Inventeur: Seuron, Patrick, F-69450 St. Cyr Au Mont d'Or (FR); Varraillon, Daniel, F-01600 Reyrieux (FR)
(74) Mandataire: Bourgouin, André

(56) Documents cités:
- EP-A- 0 091 596
- CH-A- 166 004
- FR-A- 2 329 276
- US-A- 2 759 002
- US-A- 3 134 663

## Description

La présente invention concerne un nouveau procédé de préparation de dérivés de la 1-phényl imidazoline 2,5-dione.

La présente invention a pour objet un nouveau procédé de préparation des produits de formule (I) : 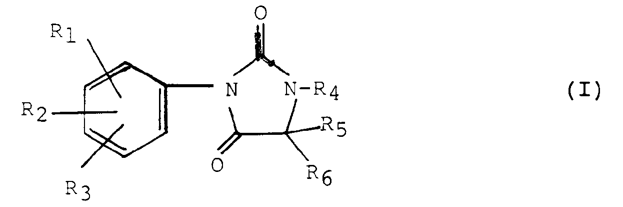
dans laquelle :
R₁, R₂ et R₃, identiques ou différents, représentent un atome d'hydrogène ou un radical alkyle, alkényle, alkynyle, alkyloxy, alkényloxy ou alkynyloxy, ces radicaux étant linéaires ou ramifiés et renfermant au plus 7 atomes de carbone ou un radical phényle, phénoxy, nitro, trifluorométhyle, acyle renfermant au plus 7 atomes de carbone ou un radical carboxy estérifié,
R₄, R₅ et R₆, identiques ou différents, représentent un atome d'hydrogène ou un radical alkyle linéaire ou ramifié renfermant au plus 7 atomes de carbone, caractérisé en ce que l'on fait réagir le produit de formule (II) : 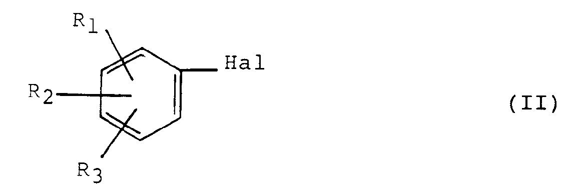
dans laquelle R₁, R₂ et R₃ ont la signification indiquée cidessus et Hal représente un atome d'halogène, avec le produit de formule (III) : 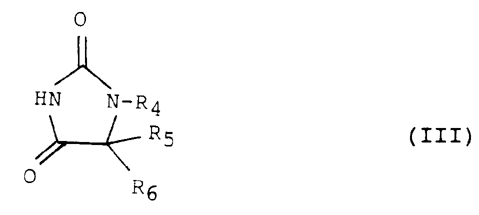
dans laquelle R₄, R₅ et R₆ ont la signification indiquée ci-dessus, la réaction s'effectuant en présence d'un catalyseur et éventuellement d'un solvant.

Dans les produits de formule (I) et dans ce qui suit :
- le terme radical alkyle linéaire ou ramifié désigne de préférence les radicaux méthyle, éthyle, n-propyle ou isopropyle, mais peut également représenter un radical n-butyle, isobutyle, sec-butyle, t-butyle ou n-pentyle,
- le terme radical alkényle linéaire ou ramifié désigne de préférence un radical vinyle, allyle, 1-propényle, butényle ou pentényle,
- le terme radical alkynyle linéaire ou ramifié désigne de préférence un radical éthynyle, propargyle, butynyle ou pentynyle,
- le terme radical alkyloxy linéaire ou ramifié désigne de préférence les radicaux méthoxy, éthoxy, n-propyloxy ou isopropyloxy, mais peut également représenter un radical butyloxy linéaire, secondaire ou tertiaire ou un radical n-pentyloxy,
- le terme radical alkényloxy linéaire ou ramifié désigne de préférence un radical allyloxy, 1-butényloxy ou pentényloxy,
- le terme radical alkynyloxy linéaire ou ramifié désigne de préférence un radical propargyloxy, butynyloxy ou pentynyloxy.

Les radicaux acyles que peuvent représenter R₁, R₂ et R₃ peuvent notamment être représentés par la formule : 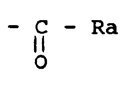
dans laquelle Ra représente un radical alkyle linéaire ou ramifié renfermant au plus 6 atomes de carbone ou un radical phényle. Les radicaux carboxy estérifiés que peuvent représenter R₁, R₂ et R₃ peuvent notamment être représentés par la formule : 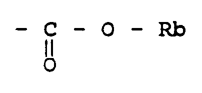
dans laquelle Rb représente un radical alkyle linéaire ou ramifié renfermant au plus 6 atomes de carbone.

Dans les deux cas les radicaux alkyle peuvent prendre les valeurs indiquées ci-dessus.
- le terme radical acyle ayant renfermant au plus 7 atomes de carbone désigne de préférence un radical acétyle, propionyle, butyryle ou benzoyle, mais également un radical valéryle, hexanoyle, acryloyle, crotonoyle ou carbamoyle,
- le terme radical carboxy estérifié désigne de préférence un groupe alkyloxy inférieur carbonyle tel que méthoxycarbonyle, éthoxycarbonyle ou aryloxycarbonyle tel que benzyloxycarbonyle.

La présente invention a tout particulièrement pour objet le procédé de préparation, tel que défini ci-dessus, du produit de formule (I) dans laquelle l'un des substituants R₁, R₂ et R₃ représente un atome d'hydrogène et les deux autres représentent un radical trifluorométhyle et un radical nitro respectivement en position 3 et en position 4 sur le cycle phényle,
R₄ représente un atome d'hydrogène et R₅ et R₆, identiques, représentent un radical méthyle.

Un procédé de préparation de ce composé qui est connu sous la Dénomination Commune Internationale NILUTAMIDE et la marque ANANDRON, est décrit dans le brevet français 2 329 276. En ce qui concerne les produits de formule (II), le terme Hal désigne de préférence un atome de chlore, mais peut aussi représenter un atome de brome ou d'iode.

Le rôle du catalyseur est vraisemblablement de piéger l'halogénure d'hydrogène qui se dégage et ainsi de faciliter la réaction de condensation du produit de formule (II) avec le produit de formule (III) pour donner le produit recherché.

L'invention a plus précisément pour objet un procédé tel que défini ci-dessus dans lequel le catalyseur est un métal sous forme native ou oxydée ou une base.

Le catalyseur utilisé peut être un métal sous forme native, sous forme d'oxyde métallique ou encore sous forme de sels métalliques. Le catalyseur peut également être une base. Quand le catalyseur utilisé est un métal, ce métal peut être du cuivre ou du nickel.

Les sels métalliques peuvent être un chlorure ou un acétate.

Quand le catalyseur est une base, cette base peut être par exemple la soude ou la potasse.

Quand le catalyseur utilisé est une base, du diméthylsulfoxyde peut, si désiré, être ajouté au milieu réactionnel.

L'invention a plus précisément pour objet un procédé tel que défini ci-dessus dans lequel le catalyseur est choisi parmi l'oxyde cuivreux, l'oxyde cuivrique, le cuivre sous forme native et une base telle que la soude ou la potasse.

Le cuivre sous forme native utilisé comme catalyseur est préférentiellement sous forme de poudre.

L'invention a particulièrement pour objet un procédé tel que défini ci-dessus dans lequel le catalyseur est l'oxyde cuivreux.

Le solvant utilisé est préférentiellement choisi parmi des éthers à haut point d'ébullition tels que, par exemple, l'oxyde de phényle, le diglyme, le triglyme et le diméthylsulfoxyde mais peut être également, par exemple, une huile à haut point d'ébullition telle que la paraffine ou la vaseline.

L'invention a plus particulièrement pour objet un procédé tel que défini ci-dessus caractérisé en ce que l'on opère en présence d'un solvant de type éther tel que l'oxyde de phényle, le diglyme, le triglyme ou le diméthylsulfoxyde.

L'invention a tout particulièrement pour objet un procédé tel que défini ci-dessus dans lequel le solvant utilisé est l'oxyde de phényle ou le triglyme.

Le procédé de préparation du produit recherché défini cidessus peut être réalisé sous pression ou à la pression atmosphérique, à une température préférentiellement élevée.

L'invention a ainsi pour objet un procédé tel que défini ci-dessus caractérisé en ce que la réaction est réalisée à une température supérieure à 100°C et de préférence supérieure à 150°C.

L'invention a plus précisément pour objet un procédé tel que défini ci-dessus caractérisé en ce que la réaction est réalisée pendant plus de 2 heures.

L'invention a très précisément pour objet un procédé tel que défini ci-dessus caractérisé en ce que la réaction est réalisée en présence d'oxyde cuivreux, dans le triglyme, à une température supérieure ou égale à 200°C et pendant plus de 3 heures.

Les produits de départ de formules (II) et (III), sur lesquels s'exerce le procédé, objet de l'invention, pour l'obtention des produits de formule (I), sont connus et disponibles dans le commerce ou peuvent être préparés selon des méthodes connues de l'homme de métier.

La préparation de produits de fromule (III) est décrite notamment dans les publications suivantes :
- Zhur. Préklad. Khim. 28, 969-75 (1955) (CA 50, 4881a, 1956)
- Tétrahédron 43, 1753 (1987)
- J. Org. 52, 2407 (1987)
- Zh. Org. Khim. 21, 2006 (1985)
- J. Fluor. Chem. 17, 345 (1981)

ou dans les brevets :
- allemand DRP 637.318 (1935)
- européen EP 0.130.875
- japonais JP 81.121.524.

Les produits de formule (III) qui sont des dérivés de l'hydantoïne sont largement utilisés et cités dans la littérature comme par exemple dans les articles suivants :
- J. Pharm. Pharmacol., 67, Vol. 19 (4), p. 209-16 (1967)
- J. Chem. Soc., 74, (2), p. 219-21 (1972)
- Khim. Farm. Zh., 67, Vol. 1 (5) p. 51-2
- Brevet allemand 2.217.914
- Brevet européen 0.091.596
- J. Chem. Soc. Perkin. Trans. 1, 74 (2) p. 48, p. 219-21.

Les exemples donnés ci-aprés illustrent l'invention sans toutefois la limiter.

### Exemple 1 : 1-(3′-trifluorométhyl 4′-nitrophényl) 4,4-diméthyl imidazoline 2,5-dione

Dans 383,52 millilitres d'oxyde de phényle, on introduit :
225, 60 grammes de 2-nitro 5-chloro trifluorométhylbenzène, décrit par exemple dans le brevet allemand DRP 637-318 (1935) et commercialisé par Hoechst ^{R},
128, 10 grammes de 5,5-diméthyl hydantoïne décrit par exemple dans Beil 24, 289 ou Fieser 7, 126 et commercialisé par Aldrich ^{R},
et 198, 53 grammes d'oxyde cuivreux

Le mélange est porté à 200°C pendant 24 heures puis refroidi à 20°C et filtré. Le résidu obtenu est rincé à l'oxyde de phényle puis extrait à l'acétate d'éthyle. La phase acétate d'éthyle est concentrée à sec sous pression réduite à 60°C puis reprise par du dichloroéthane ammoniaqué et les cristaux obtenus sont séchés à 60°C pour donner 66, 55 grammes de produit brut qui, aprés purification dans l'éthanol aqueux, donne 62, 55 grammes de produit purifié.

### Exemple 2 : 1-(3′-trifluorométhyl 4′-nitrophényl) 4,4-diméthyl imidazoline 2,5-dione

Dans 282 millilitres de triglyme, on introduit :
112, 8 grammes de 2-nitro 5-chloro trifluorométhylbenzène,
64, 1 grammes de 5,5-diméthyl hydantoïne
et 33, 5 grammes d'oxyde cuivreux.

Le mélange est porté à environ 215°C ± 5°C pendant 4 heures puis refroidi à 20°C et filtré. La solution triglyme est récupérée et à cette solution de triglyme (1V) sont ajoutés de l'ammoniaque 22 Bé (1V), du toluéne (1V) et de l'eau déminéralisée (4V), cette solution est alors agitée à 20°C pendant 15 minutes, puis refroidie à environ -10°C et agitée de nouveau à -10°C. Aprés lavage et séchage, on obtient 47, 6 grammes du produit recherché.

### Exemple 3 : 1-(3′-trifluorométhyl 4′-nitrophényl) 4,4-diméthyl imidazoline 2,5-dione

Dans 100 millilitres de diméthylsulfoxyde, 12, 80 grammes de 5, 5-diméthyl hydantoïne et 6, 28 grammes de potasse sous forme d'écailles, on introduit à 20°C sous agitation :
30 millilitres de diméthylsulfoxyde, et 24, 8 grammes de 2-nitro 5-chloro trifluorométhylbenzène.

Le mélange est porté à 110°C pendant un temps variable de 3 à 18 heures.

Le produit est caractérisé et dosé par chromatographie sur couche mince.

### Exemple 4 : 1-(3′-trifluorométhyl 4′-nitrophényl) 4,4-diméthyl imidazoline 2,5-dione

A 96,10 grammes de 5,5-diméthyl hydantoïne et 170, 86 grammes de 2-nitro 5-chloro trifluorométhylbenzène, sont ajoutés 71, 5 grammes de cuivre sous forme de poudre.

Le mélange est porté à 200°C pendant environ 21 heures, la pression étant maintenue à 450 millibares puis refroidi à 20°C et repris dans 480 millilitres d'éthanol.

Le produit est caractérisé et dosé par chromatographie sur couche mince de la solution éthanolique.

### Exemple 5 : 1-(3′-trifluorométhyl 4′-nitrophényl) 4,4-diméthyl imidazoline 2,5-dione

Dans 288 millilitres d'oxyde de phényle, on introduit :
96, 10 grammes de 5,5-diméthyl hydantoïne,
170, 86 grammes de 2-nitro 5-chloro trifluorométhylbenzène, et 89, 40 grammes d'oxyde cuivrique.

Le mélange est porté à 190°C pendant environ 23 heures puis refroidi à 20°C et filtré.

Le résidu obtenu est caractérisé dans le filtrat d'oxyde de phényle par chromatographie sur couche mince.

Les résultats analytiques obtenus pour ces 5 exemples sont identiques à ceux obtenus et indiqués dans le brevet français 2 329 276.

## Revendications

1. Nouveau procédé de préparation des produits de formule (I) : 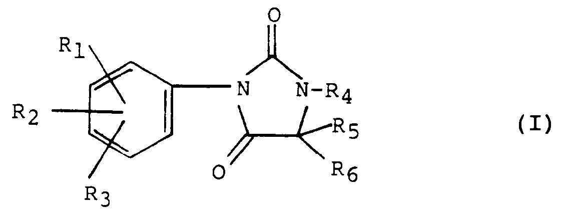 dans laquelle :
R₁, R₂ et R₃, identiques ou différents, représentent un atome d'hydrogène ou un radical alkyle, alkényle, alkynyle, alkyloxy, alkényloxy ou alkynyloxy, ces radicaux étant linéaires ou ramifiés et renfermant au plus 7 atomes de carbone ou un radical phényle, phénoxy, nitro, trifluorométhyle, acyle renfermant au plus 7 atomes de carbone ou carboxy estérifié, R₄, R₅ et R₆, identiques ou différents, représentent un atome d'hydrogène ou un radical alkyle linéaire ou ramifié, renfermant au plus 7 atomes de carbone, caractérisé en ce que l'on fait réagir le produit de formule (II) : 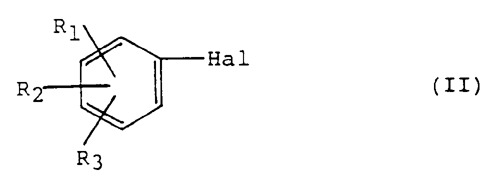 dans laquelle R₁, R₂ et R₃ ont la signification indiquée ci-dessus et Hal représente un atome d'halogène, avec le produit de formule (III) : 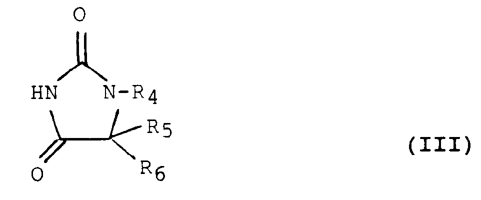 dans laquelle R₄, R₅ et R₆ ont la signification indiquée ci-dessus, la réaction s'effectuant en présence d'un catalyseur et éventuellement d'un solvant.

2. Procédé de préparation, selon la revendication 1, des produits de formule (I) dans laquelle l'un des substituants R₁, R₂ et R₃ représente un atome d'hydrogène et les deux autres représentent un radical trifluorométhyle et un radical nitro respectivement en position 3 et en position 4 sur le cycle phényle,
R₄ représente un atome d'hydrogène et R₅ et R₆, identiques, représentent un radical méthyle.

3. Procédé tel que défini à la revendication 1 ou 2 dans lequel le catalyseur est un métal sous forme native ou oxydée ou une base.

4. Procédé tel que défini aux revendications 1 à 3 dans lequel le catalyseur est choisi parmi l'oxyde cuivreux, l'oxyde cuivrique, le cuivre sous forme native et une base telle que la soude ou la potasse.

5. Procédé tel que défini aux revendications 1 à 4 dans lequel le catalyseur est l'oxyde cuivreux.

6. Procédé tel que défini aux revendications 1 à 5 caractérisé en ce que l'on opère en présence d'un solvant de type éther tel que l'oxyde de phényle, le diglyme, le triglyme ou le diméthylsulfoxyde.

7. Procédé tel que défini aux revendications 1 à 6 dans lequel le solvant utilisé est l'oxyde de phényle ou le triglyme.

8. Procédé tel que défini aux revendications 1 à 7 caractérisé en ce que la réaction est réalisée à une température supérieure à 100°C et de préférence supérieure à 150°C.

9. Procédé tel que défini aux revendications 1 à 8 caractérisé en ce que la réaction est réalisée pendant plus de 2 heures.

10. Procédé tel que défini aux revendications 1 et 2 caractérisé en ce que la réaction est réalisée en présence d'oxyde cuivreux, dans le triglyme, à une température supérieure ou égale à 200°C et pendant plus de 3 heures.

## Patentansprüche

1. Neues Verfahren zur Herstellung der Produkte der Formel (I) 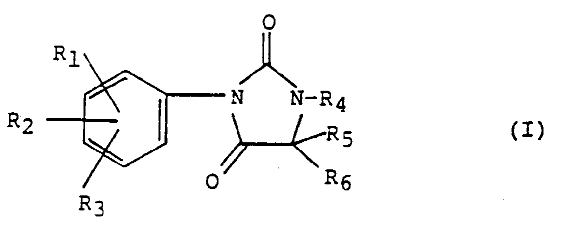 worin:
R₁, R₂ und R₃, die identisch oder verschieden sind, ein Wasserstoffatom oder einen Alkyl-, Alkenyl-, Alkinyl-, Alkyloxy-, Alkenyloxy- oder Alkinyloxyrest bedeuten, wobei diese Reste gerade oder verzweigte sind und höchstens 7 Kohlenstoffatome enthalten, oder einen Phenyl-, Phenoxy-, Nitro-, Trifluormethyl-, Acylrest mit höchstens 7 Kohlenstoffatomen oder einen veresterten Carboxyrest bedeuten, R₄, R₅ und R₆, die identisch oder verschieden sind, ein Wasserstoffatom oder einen geraden oder verzweigten Alkylrest mit höchstens 7 Kohlenstoffatomen bedeuten, dadurch gekennzeichnet, daß man das Produkt der Formel (II) 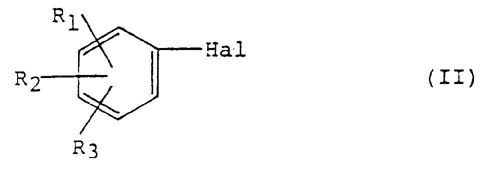 worin R₁, R₂ und R₃ die oben angegebene Bedeutung haben und Ha1 ein Halogenatom bedeutet, mit dem Produkt der Formel (III) 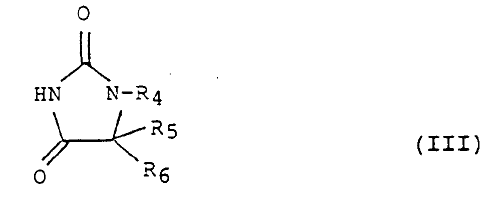 worin R₄, R₅ und R₆ die oben angegebene Bedeutung haben, zur Reaktion bringt, wobei die Reaktion in Gegenwart eines Katalysators und gegebenenfalls eines Lösungsmittels durchgeführt wird.

2. Verfahren gemäß Anspruch 1 zur Herstellung der Produkte der Formel (I), worin einer der Substituenten R₁, R₂ und R₃ ein Wasserstoffatom bedeutet und die zwei anderen einen Trifluormethyl- bzw. einen Nitrorest in 3-Stellung und 4-Stellung am Phenylring bedeuten,
R₄ ein Wasserstoffatom bedeutet, und R₅ und R₆, die identisch sind, einen Methylrest darstellen.

3. Verfahren wie in den Ansprüchen 1 oder 2 definiert, worin der Katalysator ein Metall in nativer oder oxidierter Form oder eine Base ist.

4. Verfahren wie in den Ansprüchen 1 bis 3 definiert, worin der Katalysator ausgewählt ist unter Cuprooxid, Cuprioxid, Kupfer in nativer Form und einer Base wie Natriumhydroxid oder Kaliumhydroxid.

5. Verfahren wie in den Ansprüchen 1 bis 4 definiert, worin der Katalysator ein Cuprooxid ist.

6. Verfahren wie in den Ansprüchen 1 bis 5 definiert, dadurch gekennzeichnet, daß man in Gegenwart eines Lösungsmittels vom Ethertyp arbeitet wie Phenyloxid (Phenylether), Diglyme, Triglyme oder Dimethylsulfoxid.

7. Verfahren wie in den Ansprüchen 1 bis 6 definiert, worin das verwendete Lösungsmittel Phenylether oder Triglyme ist.

8. Verfahren wie in den Ansprüchen 1 bis 7 definiert, dadurch gekennzeichnet, daß die Reaktion bei einer Temperatur oberhalb von 100 °C und vorzugsweise oberhalb von 150 °C durchgeführt wird.

9. Verfahren wie in den Ansprüchen 1 bis 8 definiert, dadurch gekennzeichnet, daß die Reaktion während mehr als 2 Stunden durchgeführt wird.

10. Verfahren wie in den Ansprüchen 1 und 2 definiert, dadurch gekennzeichnet, daß die Reaktion in Gegenwart von Cuprooxid in Triglyme bei einer Temperatur oberhalb von oder gleich 200 °C während mehr als 3 Stunden durchgeführt wird.

## Claims

1. New preparation process for products of formula (I): 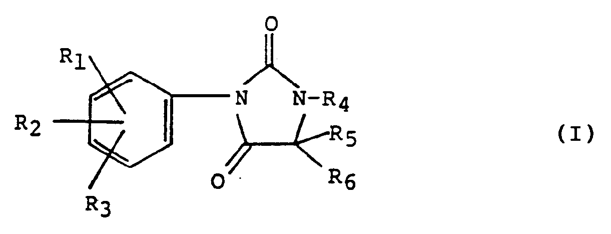 in which:
R₁, R₂ and R₃, identical or different, represent a hydrogen atom or an alkyl, alkenyl, alkynyl, alkyloxy, alkenyloxy or alkynyloxy radical, these radicals being linear or branched and containing at most 7 carbon atoms, or a phenyl, phenoxy, nitro, trifluoromethyl, acyl radical containing at most 7 carbon atoms or an esterified carboxy radical,
R₄, R₅ and R₆, identical or different, represent a hydrogen atom or a linear or branched alkyl radical containing at most 7 carbon atoms, characterized in that the product of formula (II): 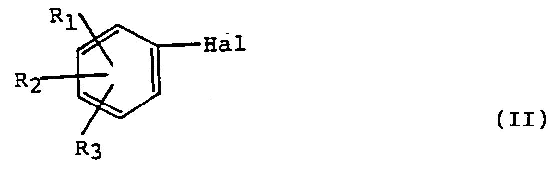 in which R₁, R₂ and R₃ have the meaning indicated above and Hal represents a halogen atom, is reacted with the product of formula (III): 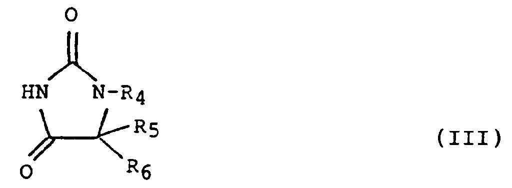 in which R₄, R₅ and R₆ have the meaning indicated above, the reaction taking place in the presence of a catalyst and optionally a solvent.

2. Preparation process, according to claim 1, for products of formula (I) in which one of the substituents R₁, R₂ and R₃ represents a hydrogen atom and the other two represent a trifluoromethyl radical and a nitro radical respectively in position 3 and in position 4 on the phenyl ring,
R₄ represents a hydrogen atom and R₅ and R₆, being identical, represent a methyl radical.

3. Process as defined in claim 1 or 2 in which the catalyst is a metal in native or oxidized form or a base.

4. Process as defined in claims 1 to 3 in which the catalyst is chosen from cuprous oxide, cupric oxide, copper in native form and a base such as soda or potash.

5. Process as defined in claims 1 to 4 in which the catalyst is cuprous oxide.

6. Process as defined in claims 1 to 5 characterized in that the operation is carried out in the presence of a solvent of ether type such as phenyl oxide, diglyme, triglyme or dimethylsulphoxide.

7. Process as defined in claims 1 to 6 in which the solvent used is phenyl oxide or triglyme.

8. Process as defined in claims 1 to 7 characterized in that the reaction is carried out at a temperature above 100°C and preferably above 150°C.

9. Process as defined in claims 1 to 8 characterized in that the reaction is carried out for more than 2 hours.

10. Process as defined in claims 1 and 2 characterized in that the reaction is carried out in the presence of cuprous oxide, in triglyme, at a temperature above or equal to 200°C and for more than 3 hours.
